# EUROPEAN PATENT SPECIFICATION

(11) **EP 1 653 880 B1**
(45) Date of publication and mention of the grant of the patent: **29.04.2009**
(21) Application number: 03749045.5
(22) Date of filing: 14.08.2003
(51) Int. Cl.: A61F 2/00

(54) **MEDICAL SLING**
MEDIZINISCHE SCHLINGE
ECHARPE MEDICALE

(43) Date of publication of application: 10.05.2006
(73) Proprietor: Boston Scientific Limited, St. Michael (BB)
(72) Inventor: CHU, Michael, S., H., Brookline, MA 02446 (US)
(74) Representative: Peterreins, Frank
(86) International application number: PCT/US2003/025452
(87) International publication number: WO 2005/018494

(56) References cited:
- WO-A-00/74633
- WO-A-98/35632
- WO-A-02/071931
- DE-U- 20 204 669
- US-A- 6 110 101
- US-A1- 2002 099 258
- US-A1- 2003 065 402

## Description

### Technical Field

This invention generally relates to treating or reinforcing a damaged, prolapsed, weakened or herniated portion of a patient's body with a sling.

### Background of the Invention

Conditions such as rectocele, cystocele, enterocele, vaginal prolapse, and protocele involve tissues or organs that have been damaged, prolapsed, weakened, or otherwise herniated. A prolapse refers to the slipping of an organ, or organ part, from its normal position. For example, a prolapse of the rectum refers to the protrusion of the rectum through the anus. Rectocele is the prolapse of the rectum into the perineum. A prolapse of the uterus refers to the falling of the uterus into the vagina due to stretching and laxity of its supporting structures. Vaginal vault prolapse refers to the prolapse of the cephalad extreme of the vaginal wall toward, through, and beyond the introitus. Cystocele (i.e., vesicocele) is a hernia formed by the downward and backward displacement of the urinary bladder toward the vaginal orifice, due most commonly to weakening of the musculature during childbirth. However, any abnormal descent of the anterior vaginal wall and bladder base at rest or with strain is considered cystocele. Enterocele is a hernia of the intestine, though the term is also used to refer specifically to herniation of the pelvic peritoneum through the rectouterine pouch (i.e., posterior vaginal, rectovaginal, cul-de-sac, or Douglas' pouch hernia). Proctocele is a prolapse of the mucous coat of the rectum due mostly from relaxation of the sphincter. Treatment of these conditions frequently requires a sling, such as a mesh sling, implanted at the anatomical site-requiring repair.

Stress urinary incontinence (SUI) primarily affects women and generally is caused by two conditions that may occur independently or in combination, namely, intrinsic sphincter deficiency (ISD) and hypermobility. In ISD, the urinary sphincter valve, located within the urethra, fails to close properly, causing urine to leak out of the urethra during stressful actions. Hypermobility is a condition in which the pelvic floor is distended, weakened, or damaged, causing the bladder neck and proximal urethra to rotate and descend in response to increases in intra-abdominal pressure (e.g., due to sneezing, coughing, straining, etc.), resulting in insufficient response time to promote urethral closure and, consequently, in urine leakage and/or flow.

Biological factors that may affect hypermobility include: poor endopelvic fascia muscle tone (from, for example, age or limited activity), endopelvic fascia muscle stretch/tear from trauma (e.g., childbirth), endopelvic fascia/arcus tendenious (muscle/ligament) separation (lateral deflect), hormone (e.g., estrogen) deficiency, concombinant defects (e.g., cystocele, enterocele, and ureteral prolapse), and vaginal prolapse. Traditional treatment methods include urethra or bladder neck stabilization slings in which a sling is placed under the mid-urethra or bladder neck to provide a platform preventing over distention.

Slings are traditionally placed under the urethra or bladder neck to provide a urethral platform limiting endopelvic fascia drop while providing compression to the urethral sphincter to improve coaptation. The urethral placement location provides mechanical stability to a less moveable anatomical structure. Bladder neck slings traditionally have been affixed in the desired location using a bone anchoring method. Mid-urethral slings, being placed in a low mobility area, may be placed using an anchorless approach. Recognizing that minimal tension, if any, is necessary, a physician may need only to secure a mid-urethra sling through the endopelvic fascia. The sling in this placement provides a fulcrum about which the pelvic floor will drop (taking advantage of the hypermobility condition of the patient) and a urethral "kink" or higher resistance to obstruct urine flow during high stress conditions.

A problem associated with sling placement is that a sling often loses its desired shape during the handling of the sling as it is implanted in a patient's body. This is due to the tension and other forces that are applied to the sling during the implantation procedure. As a result of the forces, the sling can become stretched and narrowed and may no longer be able to anchor properly in the body. This improper anchoring can result in the sling providing only poor support, leading to a failed surgical procedure.

US 6110101 discloses a bladder sling for supporting the urethra and neck of the bladder to prevent urinary incontinence, whereas the material from which the sling is made is elastic.

### Summary of the Invention

The invention, in one embodiment, addresses the deficiencies of the prior art, by providing a medical sling that maintains its shape during placement, and related methods. The invention features a sling and a method of making a sling as defined in the claims.

The invention features a sling having a first portion, a second portion and an intermediate portion, located longitudinally between the first and second portions. A first elongated member extending longitudinally along the first portion and a second elongated member extending longitudinally along the second portion; and the first and second elongated members being separated by the intermediate portion of the sling. The first and second elongated members are formed from a substantially rigid material. The intermediate portion can be of any length, e.g., the intermediate portion is between, e.g., 1 cm to 15 cm in length or 1 cm to 7 cm in length.

In another embodiment, the sling further includes a tensioning-device device located in the intermediate portion of the sling. The tensioning device can include a looped thread. The looped thread can be attached to the first elongated member, the second elongated member or both elongated members. Optionally, the tensioning device is only secured to the intermediate portion of the sling, but does not attach to the first or second elongate members. In another configuration, the thread secures to the sling material in a zigzag configuration.

In another embodiment, the tensioning device includes a handle for positioning the sling in a patent's body.

The sling can be made of various materials. In one embodiment, the sling can be made from a perforated material. In another embodiment, the sling can be made from a non-perforated material. In still yet another embodiment, the sling is a mesh. Other materials include, for example, a synthetic material, a mammalian tissue, or a combination of a synthetic material and a mammalian tissue. The sling can be of any shape suitable for its application. In a preferred configuration, the sling has a substantially rectangular shape.

In another aspect, the invention features a sling having a first edge that is structurally strengthened to increase sling rigidity. In one embodiment, the sling includes a second, structurally strengthened edge disposed opposite and away from the first edge. In another embodiment, the first edge and the second edge are detanged. According to some configurations, the sling has an irregular surface. Such irregularity may include, for example, the surface ridges, projections, depressions or combinations thereof or the like.

The invention features a method of making of making a sling as defined in the claims for implantation at an anatomical site in a patient. The method includes securing to the sling a first elongated member extending longitudinally along a first portion of the sling; securing to the sling a second elongated member extending longitudinally along a second portion of the sling, the first and second portions being separated by an intermediate portion. The first and second elongated members are made of a substantially rigid material. In one embodiment, the intermediate portion is about 1 cm to about 15 cm in length, for example, between about 1 cm to about 7 cm in length. In one embodiment, the method can further include securing a tensioning device such as a looped thread to the intermediate portion of the sling.

In another aspect, the method of the invention includes providing a sling with first and second edges and treating at least one of the edges to increase structural rigidity. The treating can include smoothing such as heating at least one of the edges. The melting can be performed with a heating element. In one embodiment, at least one of the edges includes tangs projecting therefrom and heating includes smoothing the at least one edge to substantially remove the tangs. The method further includes smoothing tangs projecting from the first edge and the second edge.

The sling can be made of various materials. In one embodiment, the sling can be made from a perforated material. In another embodiment, the sling can be made from a non-perforated material. In still yet another embodiment, the sling is a mesh. Other materials include, for example, a synthetic material, a mammalian tissue, or a combination of a synthetic material and a mammalian tissue. The sling can be of any shape suitable for its application. In a preferred configuration, the sling has a substantially rectangular shape.

In another embodiment, the invention features a method of delivering a sling to the periurethral tissues of the patient. The method includes providing a sling as described above and delivering the sling to the periurethral tissues of the patient. The method can be used, for example, to deliver a sling to a site in the body of a patient using a transvaginal, transabdominal (e.g., supra-or pre-pubic) or transobturator approach.

### Brief Description of the Drawings

In the drawings, like reference characters generally refer to the same or similar parts throughout the different views. The drawings are not necessarily to scale, but rather illustrate the principles of the invention.
FIG. 1 depicts a top view of a sling including an elongated member for inhibiting longitudinal stretching according to an illustrative embodiment of the invention.
FIG. 2 depicts a cross-sectional view of a sling including an elongated member on a first surface according to an illustrative embodiment the invention.
FIG. 3 depicts a cross-sectional view of a sling including an elongated member running through at least a portion of its length according to another illustrative embodiment the invention.
FIG. 4 depicts a top view of a sling including a plurality elongated members for inhibiting longitudinal stretching according to another illustrative embodiment of the invention.
FIG. 5 depicts a top view of a sling including a tensioning device according to an illustrative embodiment of the invention.
FIG. 6 depicts a top view of a sling including a tensioning device according to another illustrative embodiment of the invention.
FIG. 7 depicts a top view of a sling having one reinforced edge according to an illustrative embodiment of the invention.

### Illustrative Description of the Invention

In general, the invention provides a sling that overcomes many of the limitations of the slings in the prior art, which typically become misshapen during surgical placement and do not anchor properly in tissues. According to one feature, the sling of the invention has an elongated member that is attached and parallels the longitudinal axis of the sling. In addition, the sling has an intermediate portion that is free of an elongated member. The elongated member controls the longitudinal stretching of the portion of the sling to which it is attached. The elongated member thereby inhibits the portion of the sling to which it is attached from narrowing when the ends of the sling are pulled in opposite directions.

According to another feature, the sling of the invention is structurally strengthened. The structural reinforcement limits stretching and helps the sling maintain its shape.

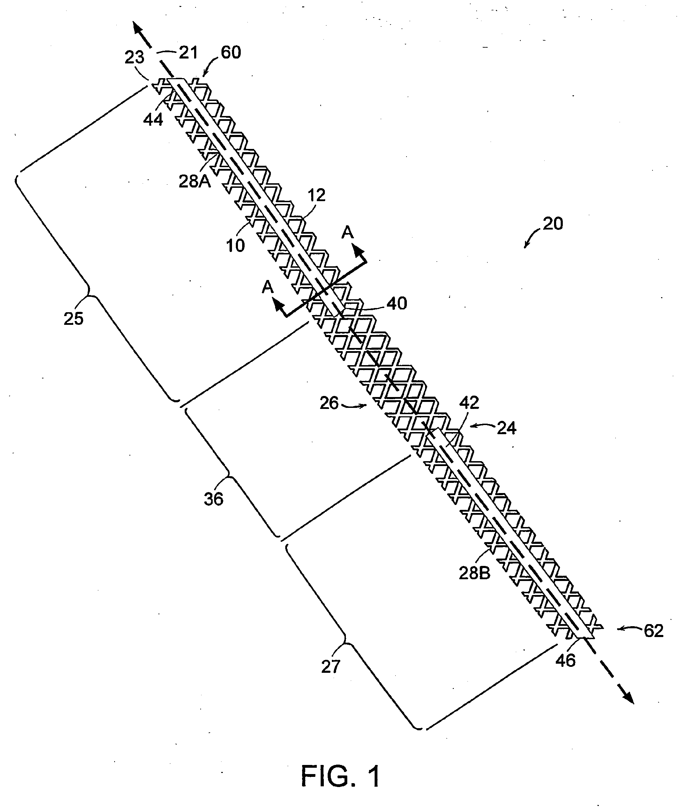

FIG. 1 depicts a top view of a sling 20 including first 28a and second 28b elongated members according to illustrative embodiment of the invention. The illustrative elongated members 28a and 28b parallel the longitudinal axis 21 of the sling 20. The sling 20 also includes first 24 and second 26 sides, first 10 and second 12 edges, and first 60 and second 62 ends. In the illustrative embodiment, the elongated members 28a and 28b are substantially rectangular and substantially flat. However, in alternative illustrative embodiments, the elongated members 28a and 28b may be suture-like.

As shown in FIG. 1, the first elongated member 28a has first 44 and second 40 terminal ends. Similarly, the second elongated member 28b has first 46 and second 42 terminal ends. Other embodiments may have more than two elongated members, for example, three, four, or five elongated members. The first elongated member 28a secures, for example, at the first end 44 to the first end 23 of the sling and longitudinally extends along a first sling portion 25. The second elongated member 28b secures, for example, at its first end 46 to the second end 62 of the sling 20 and extends longitudinally along a second sling portion 27. The first 28a and second 28b elongated members secure to the sling 20 such that the second end 40 of the first elongated member 28a and the second end 42 of the second elongated member 28b are non-overlapping.

According to this configuration, an intermediate portion 36 of the sling 20 does not include any portion of the elongated members 28a and 28b. In one particular clinical application of the illustrative embodiment of FIG. 1, the elongated member-free intermediate portion 36 of the sling 20 is implanted in close proximity to the patient's tissue in need of repair. For example, the elongated member-free intermediate portion 36 of the sling 20 may be positioned under the patient's urethra for the treatment of urinary incontinence. For this illustrative clinical application, the length of the intermediate portion 36 is preferably in a range of about 1 to about 20 cm, about 2 to about 15 cm, about 3 to about 10 cm, or about 4 to about 5 cm. So configured, the intermediate portion 36 of the sling 20 is more longitudinally expandable than the portions of the sling 20 that have an attached elongated member 28a or 28b. In other words, the size and shape of the portions of the sling 20 having an attached elongated member 28a or 28b is less changeable in its long axis in comparison to the intermediate portion 36 of the sling 20, which can expand and contract more freely along at least the longitudinal axis 21.

In one illustrative embodiment, the thickness of the sling 20, i.e., the thickness between the first side 24 and the second 26, is substantially uniform over the entire sling 20. Alternatively, the thickness varies at one or more different locations on the sling 20. In the case where the sling 20 is formed from a mesh material, thickness of the mesh may be considered, without the taking into account the holes in the mesh. The thickness of the sling 20 ranges, for example, from about 0.02 to about 0.10 cm, preferably, about 0.07 cm. The sling 20 is preferably in the range of about 20 to 50 cm in length, and about 1 to about 3 cm wide. However, the sling 20 is not limited to the size disclosed as larger or smaller slings 20 may be employed to suit various applications and the size of the patient.

According to one feature, the sling 20 is substantially rectangular in shape from a top view. In other embodiments, the sling 20 may have a trapezoidal, hexagonal, octagonal or elliptical top view shape, or any suitable shape for its intended location at an anatomical site within a patient's body. According to another feature, the sling 20 includes tangs (e.g., projections) that extend laterally from the edges 10 and 12.

The illustrative elongated members 28a and 28b secure to the sling 20, for example, by molding, gluing, bonding or weaving the elongated members 28a and 28b to the material of the sling 20, or by otherwise physically or chemically securing the elongated members 28a and 28b to the sling 20.

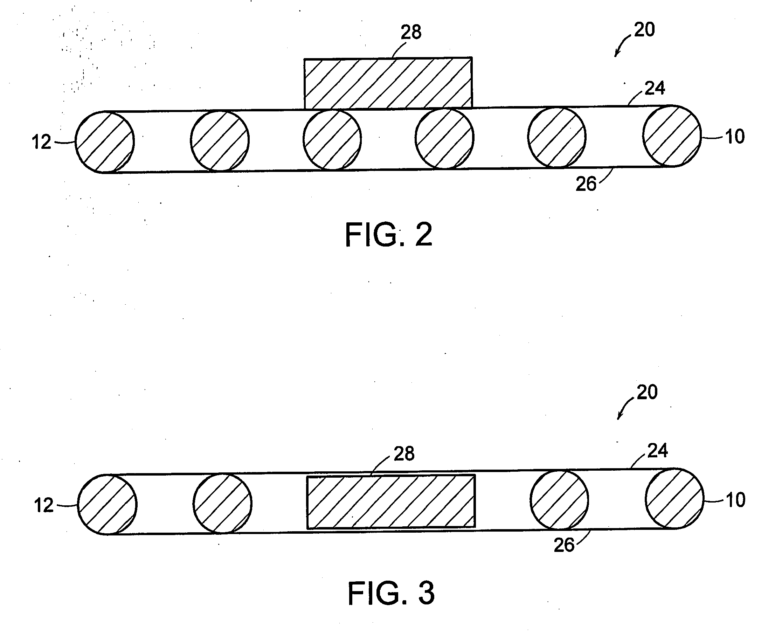

FIG. 2 depicts a cross sectional view along the line AA of the illustrative sling 20 of FIG. 1. As shown, the elongated member 28a of FIG. 2 attaches to the side 24 of the sling 20. The elongated member 28b attaches in a similar fashion. In alternative embodiments, the elongated member 28a and/or 28b may alternatively or additionally attach to the side.

FIG. 3 depicts a cross-sectional view along the line AA of a sling 20 according to an alternative illustrative embodiment of the invention. In this illustrative embodiment, the elongated members 28a and/or 28b are embedded or interwoven into the sling 20, for example, between the first side 24 and second side 26.

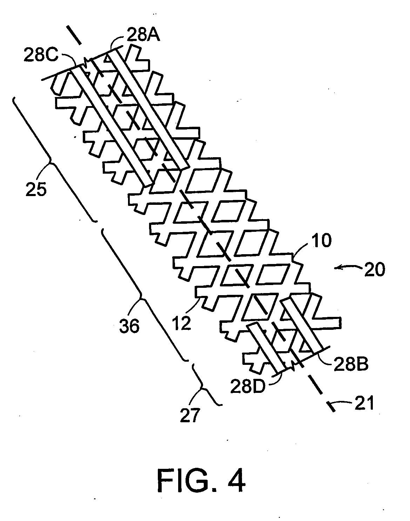

FIG. 4 depicts a top view of an alternative illustrative embodiment of the sling 20 employing four elongated members 28a-28d. In place of the single portion elongated member 28a traversing the sling 25, the embodiment of FIG. 4 provides two elongated members 28a and 28c spaced apart from each other and the edges 10 and 12, and spaced symmetrically about the central longitudinal axis 21. The two elongated members 28b and 28d are similarly spaced along the sling portion 27. As in the embodiment of FIG. 1, an intermediate portion 36 of the sling 20 is configured to be elongated member-free. Preferably, the elongated members 28a-28d are configured to be small enough so as not to substantially inhibit tissue ingrowth into the sling 20 when the sling 20 is implanted in the body of a patient.

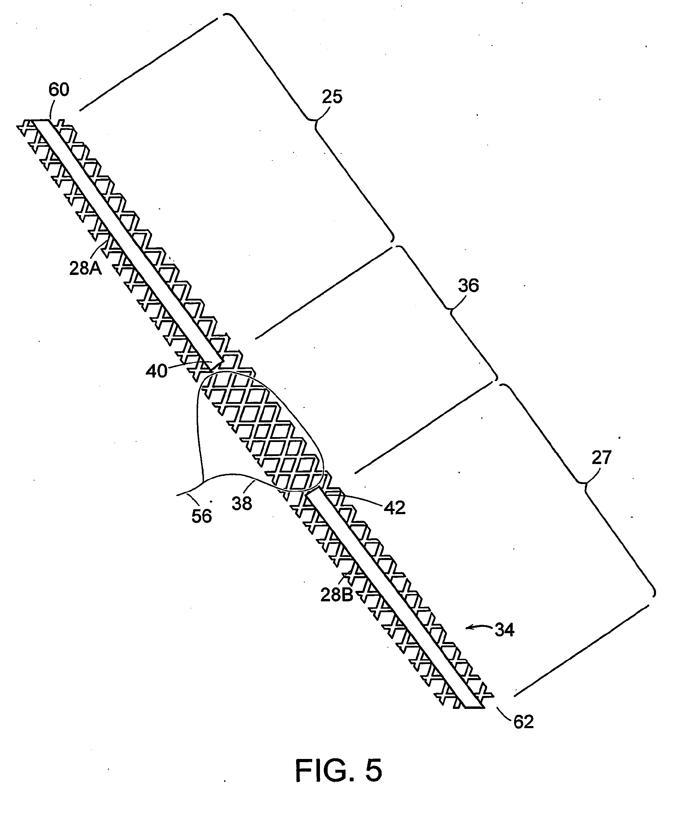

FIG. 5 depicts a top view of an alternative embodiment of the invention in which the sling 20 includes a tensioning device 38. The tensioning-device 38 limits the tension applied to the intermediate portion 36 of the sling 20. The tensioning device 38 also aids in maintaining the size and shape of the intermediate portion 36 of sling 20 during surgical placement of the sling 20 in the patient's body.

As depicted in FIG. 5, the tensioning device 38 is, for example, a suture looped to at least partially circumscribe the intermediate portion 36 of sling 20. Illustratively, the tensioning device 38 secures to the second end 40 of the first member 28a and to the second end 42 of the second member 28b. The length of the tensioning device 38 limits the length of available stretch in the intermediate portion 36 of the sling 20 when the sling 20 is pulled along its longitudinal axis 21. Preferably, the length of the tensioning device 38 is selected to prevent the intermediate portion 36 of the sling 20 from exceeding a length beyond which its elasticity is insufficient to enable it to return to its original length. According to another feature, the tensioning device 38 terminates in an end 56 configured to be used as a positioner to position the sling 20 during placement in the body of a patient. Although the end 56 is illustratively depicted as intertwined suture ends, it may have any suitable configuration such as, for example, a tab or handle.

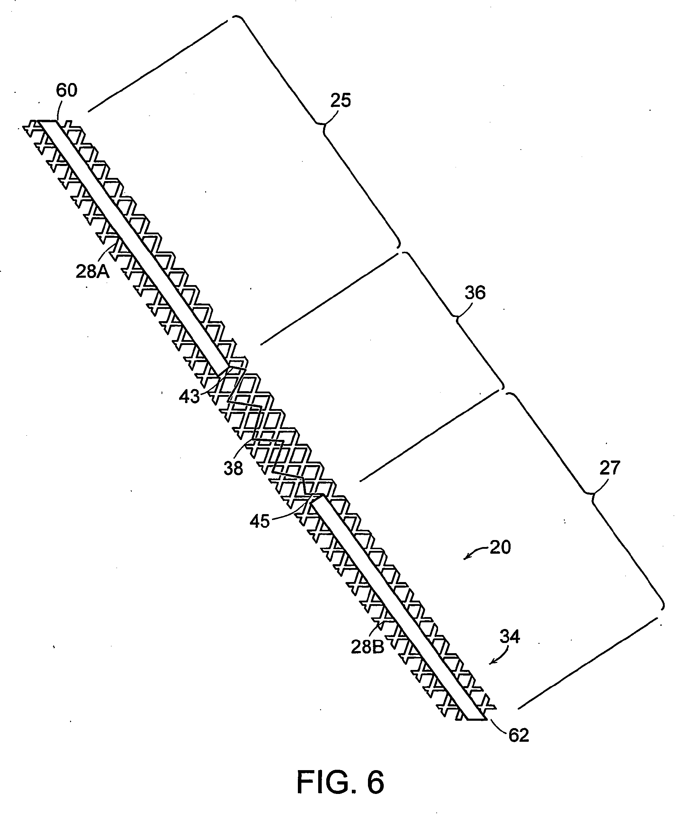

FIG. 6 depicts a top view in another alternative embodiment of the invention in which the tensioning device 38 of the sling 20 is configured as a zigzag stitch longitudinally extending along the intermediate portion 36. In this embodiment, the tensioning device, preferably, is not attached to either elongated member 28a or elongated member 28b. However, in alternative embodiments, the terminal ends 43 and 45 of the tensioning device 38 may attach to the ends 43 and 45, respectively, of the elongated elements 28a and 28b. The sling 20 may be tensed by pulling on the ends 60 and 62 of the sling 20. The amount of longitudinal expansion of the intermediate portion 36 is determined by the number length and width of the zigzag stitches.

The illustrative tensioning-device 38 of FIGS 5 and 6 may be made from, for example, resorbable or non-resorbable suture material or thread. For example, a resorbable suture material such as PLA (poly lactic acid), PGA (poly glycolic acid), PLLA (poly-l-lactic acid), or other resorbable polymers may be employed. Alternatively, non-resorbable materials, such as polypropylene (PP), polybutester or other non-resorbable polymers may be employed. According to one feature, the tensioning-device 38 is embedded into the sling 20 by, for example, weaving, molding or bonding it to the sling 20, or by otherwise physically or chemically attaching the it to the sling 20.

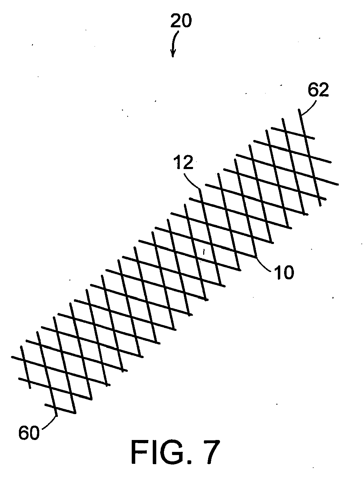

FIG. 7 is a top view of the sling 20 structurally reinforced at edge 10 but not at edge 12. Tangs (i.e., sharp projections or frayed edges) can form, for example, in the edges 10 and 12 and/or in the ends 60 and 62, when the sling material is cut, chopped, torn, frayed or otherwise manufactured. As depicted in FIG. 7, the edge 10 is smoothed to remove any such tangs and to structurally strengthen the sling 20. Any process for smoothing the edges 10 and 12 may be used. For example, the edge 10 may be heat smoothed by burning or melting. In the detanging method, the fiber ends (tangs) of the mesh are melted to a point on the mesh where the fibers cross. The melted fiber ends combine and are locked together at the intersection. Such a detanging method not only smoothes the edge 10, but also structurally stiffens it. The sling edge 12, and ends 60 and 62, may be similarly smoothed and stiffened.

An exemplary method of making a sling 20 having detanged sides 10 and 12, for example, includes manufacturing a sling material having tangs on at least side 10 or 12. The tanged sides 10 and/or 12 are then smoothed by exposing the side(s) 10 and/or 12 to a source of heat (i.e., by contact or by bringing the heat source into close proximity to the side(s) 10 and/or 12). In an alternative method, a straight blade edge that is heated to a sufficient temperature is employed simultaneously cut and smooth the side(s) 10 and/or 12.

The slings described above can be used, for example, in the treatment of urinary incontinence, and may terminate in any suitable configuration or structure such as loops, apertures, male and female connectors, guide tubes and the like. Exemplary configurations and structures are disclosed in U.S. patent application 2004-0122474 A1

In another aspect, the slings of the invention may be employed with any suitable delivery systems. Such delivery systems include, for example, those delivery systems configured for supra-pubic, pre-pubic, transvaginal or transobturator approaches. Without limitation, delivery systems and methodologies that may be employed in combination with the slings of the invention can be found, for example, in U.S. patent application 2002-015 1910 A1 U.S. patent application 2002-015 1909 A1 U.S. patent application 2002-0156489 A1 U.S. patent application 2002-0124422 A1 U.S. patent application 2003-0009 181 A1, U.S. patent application 2002-0156488 A1U.S..

As mentioned above, the slings of the invention may have any suitable size or shape configuration and may include any complimentary features. In a particular embodiment, the sling includes a protective sleeve (not shown), which encloses the sling during delivery into the patient's body. Without limitation, various applicable sling configurations are disclosed in U.S. patent application 2003-0023 137 A1 U.S. patent application 2002-015 1910 A1 U.S. patent application 2002/0138025 A1. U.S. patent application 2003-0023137 A1

In one illustrative embodiment, the sling 20 may be formed from imperforate or perforated materials. Also, the sling 20 may include a foam material. The foam material can be disposed, for example, into an interior hole or holes in the sling or otherwise embedded into the sling 20, or disposed onto one or more surfaces, sides or edges of the sling 20. The foam material can be adhered to the sling 20 by thermal bonding. The foam material can also be configured to efficiently absorb a drug or therapeutic agent prior to implantation and to release the therapeutic agent at a desired rate in the body, providing, for example, extended release. Exemplary therapeutic agent includes neomycin, sulfa drugs, antimicrobials, and antibiotics. Other exemplary therapeutic agents are described below. The foam material may be manufactured from, for example, polyvinyl acetate (PVA), polyurethane, silicone, polyester, polyethylene, etc.

The elongated members 28a, 28b, 28c and/or 28d of the invention can be made from the same materials as the sling 20 as described below. In a particular embodiment, the elongated members 28a-28d are made from materials that are more rigid than the materials used to make the sling 20. The elongated members 28a-28d may be made, for example, from resorbable or non-resorbable suture material or thread, Resorbable suture materials such as PLA (poly lactic acid), PGA (poly glycolic acid), PLLA (poly-l-lactic acid), or other resorbable polymers may be employed. Non-resorbable materials such as polypropylene (PP), polybutester or other non-resorbable polymers may be employed.

The sling 20 may be fabricated from any of a number of biocompatible materials such as nylon, polyethylene, polyester, polypropylene, fluoropolymers, copolymers thereof, combinations thereof, or other suitable synthetic material(s). The sling 20 may be, for example, a synthetic material that is absorbable by the patient's body. Suitable absorbable synthetic materials include polyglycolic acid (PGA), polylactic acid, and other suitable absorbable synthetic materials. A suitable PGA material is available under the trade designation DEXON, from TYCO (Exeter, NH). Other suitable polymeric, non-polymeric synthetic materials or their combination may be employed in accordance with the invention. In one embodiment, the synthetic material is porous.

Alternatively, the sling material may be derived from mammalian tissue(s). The mammalian tissue source may be, for example, human, human cadaveric, or tissue-engineered human tissue. The mammalian tissue may alternatively be derived from an animal source such as porcine, ovine, bovine, and equine tissue sources.

The sling material may also be made of a combination of mammalian tissue and synthetic material. Such combinations may also include materials that include both synthetic material and animal cells that are treated so as to cross-link the collagen or other commonly antigenic fibers of the animal cells. In one embodiment, at least a portion of the sling 20, which contacts the patient's tissue, comprises a synthetic material that is substantially smooth.

The sling 20 in some configurations is made of a non-wettable material such as a polypropylene, polyethylene, polyester, polytetrafluoroethylene, TYVEK available from DuPont, PA, MYLAR available from DuPont, PA, or co-polymers thereof. Polytetrafluoroethylene, which is suitable for use in accordance with the present invention, is available from DuPont (Wilmington, Delaware, under the trade designation TEFLON).

Such non-wettable materials do not take up any liquids, for example, therapeutic agents in solution. To permit therapeutic agents to bond or absorb to these non-wettable material sides, the sling 20 may be treated with a substance that is wettable such as, for example, a wettable coating composition. The wettable coating composition may be a synthetic coating (e.g., polyvinylpyrrlidone or PVP), a natural coating (e.g., collagen) or a physically absorbent material (e.g., sponge comprising cellulose or open celled polyurethane). The wettable coating composition may be hydrophilic. Suitable hydrophilic coatings may be water soluble and include, for example, such coatings available under the trade designations Hydroplus and Hydropass. Similarly, a hydrophobic coating may be employed on one or more surfaces of the sling 20. Suitable hydrophobic coatings that may be employed in accordance with the invention include but are not limited to polytetrafluoroethylene, silicon, and Pyrelene.

Therapeutic agents may also be employed with sling 20. For example, the hydrophilic coating and the therapeutic agent are mixed to form a single coating. Alternatively, the therapeutic agents may be compressed into the material of the sling, rather than being applied as a coating.

The therapeutic agents can be, for example, hydrophilic or hydrophobic. Hydrophilic drugs that may be employed in accordance with the invention include oxybutynin chloride, lidocaine, ketorolac, ketorolac tromethamine, which is available under the trade designation Toradol from Roche Pharmaceuticals (Nutley, NJ) and hyoscyamine sulfate which is available under the trade designation CYTOSPAZ from Polymedica (Woburn, MA), for example. Suitable hydrophobic drugs include but are not limited to ibuprofen, ketoprofen, and diclofenac. The drug can be mixed with the coating and applied with the coating. Where the bonding between the coatings and drugs is weak, the drug that is absorbed will readily release to be delivered to the sides it contacts. Alternatively, a stronger bonding affinity may provide a slower timed release of the drug.

Where the coating applied to the surface of the sling 20 has an ionic charge, drugs comprising a complementary charge will bond to the coating when the coating and the drug are exposed to one another. The strength of any bonding will impact how readily the drug is released from the sling 20. Where the ionic bonding between the coating and the drug is weak, the drug will release more readily. In embodiments where rapid drug release is desirable, covalent bonding between the side coating and the drug should be avoided.

In general, the therapeutic agent for use in connection with the present invention can be any pharmaceutically acceptable therapeutic agent. Preferred therapeutic agents include anti-inflammatory agents, analgesic agents, local anesthetic agents, antispasmodic agents, and combinations thereof.

Anti-inflammatory agents include steroidal and non-steroidal anti-inflammatory agents. Examples of non-steroidal anti-inflammatory drugs, include aminoarylcarboxylic acid derivatives such as enfenamic acid, etofenamate, flufenamic acid, isonixin, meclofenamic acid, mefanamic acid, niflumic acid, talniflumate, terofenamate and tolfenamic acid; arylacetic acid derivatives such as acemetacin, alclofenac, amfenac, bufexamac, cinmetacin, clopirac, diclofenac sodium, etodolac, felbinac, fenclofenac, fenclorac, fenclozic acid, fentiazac, glucametacin, ibufenac, indomethacin, isofezolac, isoxepac, lonazolac, metiazinic acid, oxametacine, proglumetacin, sulindac, tiaramide, tolmetin and zomepirac; arylbutyric acid derivatives such as bumadizon, butibufen, fenbufen and xenbucin; arylcarboxylic acids such as clidanac, ketorolac and tinoridine; arylpropionic acid derivatives such as alminoprofen, benoxaprofen, bucloxic acid, carprofen, fenoprofen, flunoxaprofen, flurbiprofen, ibuprofen, ibuproxam, indoprofen, ketoprofen, loxoprofen, miroprofen, naproxen, oxaprozin, piketoprofen, pirprofen, pranoprofen, protizinic acid, suprofen and tiaprofenic acid; pyrazoles such as difenamizole and epirizole; pyrazolones such as apazone, benzpiperylon, feprazone, mofebutazone, morazone, oxyphenbutazone, phenybutazone, pipebuzone, propyphenazone, ramifenazone, suxibuzone and thiazolinobutazone; salicylic acid derivatives such as acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamine o-acetic acid, salicylsulfuric acid, salsalate and sulfasalazine; thiazinecarboxamides such as droxicam, isoxicam, piroxicam and tenoxicam; others such as ε-acetamidocaproic acid, s-adenosylinethionine, 3-amino-4-hydroxybutyric acid, amixetrine, bendazac, benzydamine, bucolome, difenpiramide, ditazol, emorfazone, guaiazulene, nabumetone, nimesulide, orgotein, oxaceprol, paranyline, perisoxal, pifoxime, proquazone, proxazole and tenidap; and pharmaceutically acceptable salts thereof.

Examples of steroidal anti-inflammatory agents (glucocorticoids) include 21-acetoxyprefnenolone, aalclometasone, algestone, amicinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumehtasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol priopionate, halometasone, halopredone acetate, hydrocortamate, hydrocortisone, loteprednol etabonate, mazipredone, medrysone, meprednisone, methyolprednisolone, mometasone furoate, paramethasone, prednicarbate, prednisolone, prednisolone 25-diethylaminoacetate, prednisone sodium phosphate, prednisone, prednival, prednylidene, rimexolone, tixocortal, triamcinolone, triamcinolone acetonide, triamcinolone benetonide, triamcinolone hexacetonide, and pharmaceutically acceptable salts thereof.

Analgesic agents include narcotic and non-narcotic analgesics. Narcotic analgesic agents include alfentanil, allylprodine, alphaprodine, anileridine, benzylmorphine, bezitramide, buprenorphine, butorphanol, clonitazene, codeine, codeine methyl bromide, codeine phosphate, codeine sulfate, desomorphine, dextromoramide, dezocine, diampromide, dihydrocodeine, dihydrocodeinone enol acetate, dihydromorphine, dimenoxadol, dimepheptanol, dimethylthiambutene, dioxaphetyl butyrate, dipipanone, eptazocine, ethoheptazine, ethylmethlythiambutene, ethylmorphine, etonitazene, fentanyl, hydrocodone, hydromorphone, hydroxypethidine, isomethadone, ketobemidone, levorphanol, lofentanil, meperidine, meptazinol, metazocine, methadone hydrochloride, metopon, morphine, myrophine, nalbuphine, narceine, nicomorphine, norlevorphanol, normethadone, normorphine, norpipanone, opium, oxycodone, oxymorphone, papaveretum, pentazocine, phenadoxone, phenazocine, pheoperidine, piminodine, piritramide, proheptazine, promedol, properidine, propiram, propoxyphene, rumifentanil, sufentanil, tilidine, and pharmaceutically acceptable salts thereof.

Non-narcotic analgesics include aceclofenac, acetaminophen, acetaminosalol, acetanilide, acetylsalicylsalicylic acid, alclofenac, alminoprofen, aloxiprin, aluminum bis(acetylsalicylate), aminochlorthenoxazin, 2-amino-4-picoline, aminopropylon, aminopyrine, ammonium salicylate, amtolmetin guacil, antipyrine, antipyrine salicylate, antrafenine, apazone, aspirin, benorylate, benoxaprofen, benzpiperylon, benzydamine, bermoprofen, brofenac, p-bromoacetanilide, 5-bromosalicylic acid acetate, bucetin, bufexamac, bumadizon, butacetin, calcium acetylsalicylate, carbamazepine, carbiphene, carsalam, chloralantipyrine, chlorthenoxazin(e), choline salicylate, cinchophen, ciramadol, clometacin, cropropamide, crotethamide, dexoxadrol, difenamizole, diflunisal, dihydroxyaluminum acetylsalicylate, dipyrocetyl, dipyrone, emorfazone, enfenamic acid, epirizole, etersalate, ethenzamide, ethoxazene, etodolac, felbinac, fenoprofen, floctafenine, flufenamic acid, fluoresone, flupirtine, fluproquazone, flurbiprofen, fosfosal, gentisic acid, glafenine, ibufenac, imidazole salicylate, indomethacin, indoprofen, isofezolac, isoladol, isonixin, ketoprofen, ketorolac, p-lactophenetide, lefetamine, loxoprofen, lysine acetylsalicylate, magnesium acetylsalicylate, methotrimeprazine, metofoline, miroprofen, morazone, morpholine salicylate, naproxen, nefopam, nifenazone, 5' nitro-2' propoxyacetanilide, parsalmide, perisoxal, phenacetin, phenazopyridine hydrochloride, phenocoll, phenopyrazone, phenyl acetylsalicylate, phenyl salicylate, phenyramidol, pipebuzone, piperylone, prodilidine, propacetamol, propyphenazone, proxazole, quinine salicylate, ramifenazone, rimazolium metilsulfate, salacetamide, salicin, salicylamide, salicylamide o-acetic acid, salicylsulfuric acid, salsalte, salverine, simetride, sodium salicylate, sulfamipyrine, suprofen, talniflumate, tenoxicam, terofenamate, tetradrine, tinoridine, tolfenamic acid, tolpronine, tramadol, viminol, xenbucin, zomepirac, and pharmaceutically acceptable salts thereof.

Local anesthetic agents include amucaine, amolanone, amylocaine hydrochloride, benoxinate, benzocaine, betoxycaine, biphenamine, bupivacaine, butacaine, butaben, butanilicaine, butethamine, butoxycaine, carticaine, chloroprocaine hydrochloride, cocaethylene, cocaine, cyclomethycaine, dibucaine hydrochloride, dimethisoquin, dimethocaine, diperadon hydrochloride, dyclonine, ecgonidine, ecgonine, ethyl chloride, beta-eucaine, euprocin, fenalcomine, fomocaine, hexylcaine hydrochloride, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine hydrochloride, phenol, piperocaine, piridocaine, polidocanol, pramoxine, prilocaine, procaine, propanocaine, proparacaine, propipocaine, propoxycaine hydrochloride, pseudococaine, pyrrocaine, ropavacaine, salicyl alcohol, tetracaine hydrochloride, tolycaine, trimecaine, zolamine, and pharmaceutically acceptable salts thereof.

Antispasmodic agents include alibendol, ambucetamide, aminopromazine, apoatropine, bevonium methyl sulfate, bietamiverine, butaverine, butropium bromide, n-butylscopolammonium bromide, caroverine, cimetropium bromide, cinnamedrine, clebopride, coniine hydrobromide, coniine hydrochloride, cyclonium iodide, difemerine, diisopromine, dioxaphetyl butyrate, diponium bromide, drofenine, emepronium bromide, ethaverine, feclemine, fenalamide, fenoverine, fenpiprane, fenpiverinium bromide, fentonium bromide, flavoxate, flopropione, gluconic acid, guaiactamine, hydramitrazine, hymecromone, leiopyrrole, mebeverine, moxaverine, nafiverine, octamylamine, octaverine, oxybutynin chloride, pentapiperide, phenamacide hydrochloride, phloroglucinol, pinaverium bromide, piperilate, pipoxolan hydrochloride, pramiverin, prifinium bromide, properidine, propivane, propyromazine, prozapine, racefemine, rociverine, spasmolytol, stilonium iodide, sultroponium, tiemonium iodide, tiquizium bromide, tiropramide, trepibutone, tricromyl, trifolium, trimebutine, n,n-1trimethyl-3,3-diphenyl-propylamine, tropenzile, trospium chloride, xenytropium bromide, and pharmaceutically acceptable salts thereof.

Two particularly preferred therapeutic agents for the practice of the present invention are (a) ketorolac and pharmaceutically acceptable salts thereof (e.g., the tromethamine salt thereof, sold under the commercial name Toradol®) and (b) 4-diethylamino-2-butynylphenylcyclohexylglycolate and pharmaceutically acceptable salts thereof (e.g., 4-diethylamino-2-butynylphenylcyclohexylglycolate hydrochloride, also known as oxybutynin chloride, sold under the commercial name Ditropan®).

The amount of the therapeutic agent present in the polymeric matrix is an amount effective to reduce the pain or discomfort associated with the medical device. Typically, the therapeutic agent is present in a polymeric matrix in a range from about 0.1% to about 30% by weight of the polymeric matrix (including 0.1%, 0.2%, 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30% and ranges between any two of these points, for instance, 0.1-10%, 10-20% and 20-30%, etc.). Where the oxybutynin chloride and ketorolac tromethamine are used a range of 2-20% is typical, more typically 5-15%.

Alternatively, other therapeutic agents as known to those in the field as useful to enhance the efficacy of the sling or reduce adverse reactions to the sling, for example, are contemplated with respect to the invention.

An exemplary method of making a sling 20 includes constructing a length of material having a first portion that is relatively stretchable and a second portion that is substantially non-stretchable. In one embodiment, the sling 20 is manufactured having a member extending longitudinally along the second portion of the sling 20.

The sling 20 disclosed herein can be used to treat female urinary stress incontinence. Methods of sling delivery and implantation include but are not limited to tranvaginal, transabdominal, and transobturator procedures. In one example, a sling 20 having a first and a second edge structurally strengthened is placed inside the body of a patient such that the axis of the sling 20 that is perpendicular to the long axis of the sling parallels a portion of the mid-urethra. The sling 20 thereby provides a urethral platform limiting endopelvic fascia drop while providing compression to the urethral sphincter.

## Claims

1. A sling (20) comprising:
a first portion (25), a second portion (27), and an intermediate portion (36) located longitudinally between the first (25) and second (27) portions;
a first elongated member (28a) extending longitudinally along the first portion (25);
a second elongated member (28b) extending longitudinally along the second portion (27); and
the first (28a) and second (28b) elongated members being separated by the intermediate portion (36) of the sling (20); wherein
the first (28a) and second (28b) elongated members comprise a substantially rigid material.

2. The sling according to claim 1; wherein at least one of the first (28a) and second (28b) elongated members is integral with the portions of the sling along which the members extend.

3. The sling according to claim 1, wherein the intermediate portion (36) is 1 cm to 15 cm in length.

4. The sling according to claim 1, wherein the intermediate portion (36) is 1 cm to 7 cm in length.

5. The sling according to claim 1, further comprising a tensioning device (38) located in the intermediate portion (36) of the sling.

6. The sling according to claim 5, wherein the tensioning device (38) comprises a looped thread.

7. The sling according to claim 6, wherein the thread (38) is attached to one or both of the first (28a) and second (28b) elongated members.

8. The sling according to claim 1, wherein the sling (20) is non-perforated.

9. The sling according to claim 1, wherein the sling (20) is a mesh.

10. The sling according to claim 1, further comprising a first edge (10) structurally strengthened to increase sling rigidity.

11. The sling according to claim 10, comprising a second edge structurally strengthened to increase sling rigidity.

12. The sling according to claim 11, wherein at least a portion of the first edge is detanged.

13. The sling according to claim 12, wherein at least a portion of the second edge is detanged.

14. The sling according to claim 13, wherein at least a portion of the first edge and the second edge is detanged.

15. A method of making a sling (20) according to claim 1 for implantation at an anatomical site in a patient, the method comprising:
securing to the sling (20) a first elongated member (28a) extending longitudinally along a first portion (25) of the sling (20); and
securing to the sling (20) a second elongated member (28b) extending longitudinally along a second portion (27) of the sling (20), the first (25) and second (27) portions being separated by an intermediate portion (36);
wherein the first (28a) and second (28b) elongated members comprise a substantially rigid material.

16. The method according to claim 15, wherein the intermediate portion (36) is 1 cm to 15 cm in length.

17. The method according to claim 15, wherein the intermediate portion (36) is 1 cm to 7 cm in length.

18. The method according to claim 15, wherein the sling (20) is perforated.

19. The method according to claim 15, wherein the sling (20) is a mesh.

20. The method according to claim 15, wherein the sling (20) comprises a synthetic material.

21. The method according to claim 15, wherein the sling (20) comprises a mammalian tissue.

22. The method according to claim 15, wherein the sling (20) comprises a combination of a synthetic material and a mammalian tissue.

23. A method of making a sling according to claim 15, the method comprising the steps of:
providing the sling having first (10) and second (12) edges, and
treating at least a portion of at least one of the first (10) and second (12) edges to increase structural rigidity of the sling.

24. The method of claim 23, wherein the treating comprises smoothing the portion of the at least one of the first (10) and second (12) edges.

25. The method of claim 24, wherein the smoothing comprises melting the portion of the at least one of the first (10) and second (12) edges.

26. The method of claim 25, wherein the melting is performed with a heating element.

27. The method of claim 23, wherein the at least one of the first or second edges comprises tangs and treating at least the portion comprising smoothing the portion to substantially remove the tangs.

28. The method of claim 23, wherein the sling is formed from a non-perforated material.

29. The method of claim 23, wherein the sling is formed from a perforated material.

## Patentansprüche

1. Schlinge (20) umfassend:
einen ersten Bereich (25), einen zweiten Bereich (27) und einen Zwischenbereich (36), angeordnet in Längsrichtung zwischen den ersten (25) und zweiten (27) Bereichen;
ein erstes längliches Element (28a), sich erstreckend in Längsrichtung entlang des ersten Bereichs (25);
ein zweites längliches Element (28b), sich erstreckend in Längsrichtung entlang des zweiten Bereiches (27); und
wobei die ersten (28a) und zweiten (28b) länglichen Elemente durch den Zwischenbereich (36) der Schlinge (20) getrennt sind; und wobei
die ersten (28a) und zweiten (28b) länglichen Elemente ein im Wesentlichen rigides Material umfassen.

2. Schlinge nach Anspruch 1, wobei mindestens eins der ersten (28a) und zweiten (28b) länglichen Elemente integral mit den Bereichen der Schlinge, entlang welcher die Elemente sich erstrecken, ist.

3. Schlinge nach Anspruch 1, wobei der Zwischenbereich (36) 1 cm bis 15 cm lang ist.

4. Schlinge nach Anspruch 1, wobei der Zwischenbereich (36) 1 cm bis 7 cm lang ist.

5. Schlinge nach Anspruch 1, weiter umfassend eine Spannungsvorrichtung (38), angeordnet im Zwischenbereich (36) der Schlinge.

6. Schlinge nach Anspruch 5, wobei die Spannungsvorrichtung (38) einen schlaufenförmigen Faden umfasst.

7. Schlinge nach Anspruch 6, wobei der Faden (38) an einem oder beiden der ersten (28a) und zweiten (28b) länglichen Elemente befestigt ist.

8. Schlinge nach Anspruch 1, wobei die Schlinge (20) nicht perforiert ist.

9. Schlinge nach Anspruch 1, wobei die Schlinge (20) ein Netz ist.

10. Schlinge nach Anspruch 1, weiter umfassend eine erste Kante (10), strukturell verstärkt, um die Schlingenrigidität zu erhöhen.

11. Schlinge nach Anspruch 10, umfassend eine zweite Kante, strukturell verstärkt, um die Schlingenrigidität zu erhöhen.

12. Schlinge nach Anspruch 11, wobei mindestens ein Bereich der ersten Kante von scharfen Projektionen befreit ist.

13. Schlinge nach Anspruch 12, wobei mindestens ein Bereich der zweiten Kante von scharfen Projektionen befreit ist.

14. Schlinge nach Anspruch 13, wobei mindestens ein Bereich der ersten Kante und der zweiten Kante von scharfen Projektionen befreit ist.

15. Verfahren zur Herstellung einer Schlinge (20) nach Anspruch 1, zur Implantierung an einer anatomischen Stelle in einem Patienten, das Verfahren umfassend:
Sichern eines ersten länglichen Elementes (28a), sich erstreckend in Längsrichtung entlang einen ersten Bereichs (25) der Schlinge (20), an der Schlinge (20); und
Sichern eines zweiten länglichen Elementes (28b), sich erstreckend in Längsrichtung entlang eines zweiten Bereichs (27) der Schlinge (20), an der Schlinge (20), wobei die ersten (25) und zweiten (27) Bereiche durch einem Zwischenbereich (36) getrennt sind; und wobei die ersten (28a) und zweiten (28b) länglichen Elemente ein im Wesentlichen rigides Material umfassen.

16. Verfahren nach Anspruch 15, wobei der Zwischenbereich (36) 1 cm bis 15 cm lang ist.

17. Verfahren nach Anspruch 15, wobei der Zwischenbereich (36) 1 cm bis 7 cm lang ist.

18. Verfahren nach Anspruch 15, wobei die Schlinge (20) perforiert ist.

19. Verfahren nach Anspruch 15, wobei die Schlinge (20) ein Netz ist.

20. Verfahren nach Anspruch 15, wobei die Schlinge (20) ein synthetisches Material umfasst.

21. Verfahren nach Anspruch 15, wobei die Schlinge (20) ein Säugetiergewebe umfasst.

22. Verfahren nach Anspruch 15, wobei die Schlinge (20) eine Kombination eines synthetischen Materials und eines Säugetiergewebes umfasst.

23. Verfahren zur Herstellung einer Schlinge nach Anspruch 15, das Verfahren umfassend die Schritte:
Bereitstellen der Schlinge, wobei die Schlinge erste (10) und zweite (12) Kanten hat, und
Behandeln mindestens eines Bereiches von mindestens einer der ersten (10) und zweiten (12) Kanten, um strukturelle Rigidität der Schlinge zu erhöhen.

24. Verfahren nach Anspruch 23, wobei das Behandeln das Glätten des Bereichs der mindestens einen der ersten (10) und zweiten (12) Kanten umfasst.

25. Verfahren nach Anspruch 24, wobei das Glätten umfasst Schmelzen des Bereiches der mindestens einen der ersten (10) und zweiten (12) Kanten.

26. Verfahren nach Anspruch 25, wobei das Schmelzen mit einem Heizelement durchgeführt wird.

27. Verfahren nach Anspruch 23, wobei die mindestens eine der ersten oder zweiten Kanten scharfe Projektionen umfasst und Behandeln mindestens des Bereiches umfassend Glätten des Bereiches, um die spitzen Projektionen im Wesentlichen zu entfernen.

28. Verfahren nach Anspruch 23, wobei die Schlinge durch ein nicht perforiertes Material gebildet ist.

29. Verfahren nach Anspruch 23, wobei die Schlinge durch ein perforiertes Material gebildet ist.

## Revendications

1. Echarpe (20) comprenant :
une première partie (25), une deuxième partie (27), et une partie intermédiaire (36) située longitudinalement entre la première (25) et la deuxième (27) parties ;
un premier élément allongé (28a) s'étendant longitudinalement le long de la première partie (25) ;
un second élément allongé (28b) s'étendant longitudinalement le long de la deuxième partie (27) ; et
le premier (28a) et le deuxième (28b) éléments allongés étant séparés par la partie intermédiaire (36) de l'écharpe (20), dans laquelle
le premier (28a) et le deuxième (28b) éléments allongés comprennent un matériau sensiblement rigide.

2. Echarpe selon la revendication 1, dans laquelle au moins le premier (28a) ou le deuxième (28b) éléments allongés sont d'un seul tenant avec les parties de l'écharpe le long desquelles les éléments s'étendent.

3. Echarpe selon la revendication 1, dans laquelle la partie intermédiaire (36) mesure de 1 cm à 15 cm de long.

4. Echarpe selon la revendication 1, dans laquelle la partie intermédiaire (36) mesure de 1 cm à 7 cm de long.

5. Echarpe selon la revendication 1, comprenant en outre un dispositif de tension (38) situé dans la partie intermédiaire (36) de l'écharpe.

6. Echarpe selon la revendication 5, dans laquelle le dispositif de tension (38) comprend un fil à boucle.

7. Echarpe selon la revendication 6, dans laquelle le fil (38) est fixé à un ou aux deux éléments parmi le premier (28a) et le deuxième (28b) éléments allongés.

8. Echarpe selon la revendication 1, dans laquelle ladite écharpe (20) est non-perforée.

9. Echarpe selon la revendication 1, dans laquelle l'écharpe (20) est un treillis.

10. Echarpe selon la revendication 1, comprenant en outre un premier bord (10) structurellement renforcé afin d'augmenter la rigidité de l'écharpe.

11. Echarpe selon la revendication 10, comprenant un second bord structurellement renforcé pour augmenter la rigidité de l'écharpe.

12. Echarpe selon la revendication 11, dans laquelle au moins une partie du premier bord est lissée pour éliminer les fils.

13. Echarpe selon la revendication 12, dans laquelle au moins une partie du second bord est lissée pour éliminer les fils.

14. Echarpe selon la revendication 13, dans laquelle au moins une partie du premier bord et du second bord est lissée pour éliminer les fils.

15. Procédé de réalisation d'une écharpe (20) selon la revendication 1 pour implantation sur un site anatomique d'un patient, le procédé comprenant :
la fixation à l'écharpe (20) d'un premier élément allongé (28a) s'étendant longitudinalement le long d'une première partie (25) de l'écharpe (20) ; et la fixation à l'écharpe (20) d'un deuxième élément allongé (28b) s'étendant longitudinalement le long d'une deuxième partie (27) de l'écharpe (20), la première (25) et la deuxième (27) parties étant séparées par une partie intermédiaire (36) ; dans lequel le premier (28a) et le deuxième (28b) éléments allongés comprennent un matériau sensiblement rigide.

16. Procédé selon la revendication 15, dans lequel la partie intermédiaire (36) mesure de 1 cm à 15 cm de long.

17. Procédé selon la revendication 15, dans lequel la partie intermédiaire (35) mesure de 1 cm à 7 cm de long.

18. Procédé selon la revendication 15, dans lequel l'écharpe (20) est perforée.

19. Procédé selon la revendication 15, dans lequel l'écharpe (20) est un treillis.

20. Procédé selon la revendication 15, dans lequel l'écharpe (20) comprend un matériau synthétique.

21. Procédé selon la revendication 15, dans lequel l'écharpe (20) comprend un tissu de mammifère.

22. Procédé selon la revendication 15, dans lequel l'écharpe (20) comprend une combinaison d'un matériau synthétique et d'un tissu de mammifère.

23. Procédé de réalisation d'une écharpe selon la revendication 15, ledit procédé comprenant les étapes consistant à :
fournir une écharpe ayant un premier (10) et un second (12) bords, et traiter au moins une partie d'au moins le premier (10) ou le deuxième (12) bords afin d'augmenter la rigidité structurelle de l'écharpe.

24. Procédé selon la revendication 23, dans lequel le traitement comprend le lissage de la partie dudit au moins le premier (10) ou le deuxième (12) bords.

25. Procédé selon la revendication 24, dans lequel le lissage comprend la fusion de la partie dudit premier (10) ou deuxième (12) bords.

26. Procédé selon la revendication 25, dans lequel la fusion est effectuée avec un élément chauffant.

27. Procédé selon la revendication 23, dans lequel ledit premier ou ledit second bords comprend des fils et le traitement d'au moins la partie comprenant le lissage de la portion afin d'éliminer sensiblement les fils

28. Procédé selon la revendication 23, dans lequel l'écharpe est formée d'un matériau non-perforé.

29. Procédé selon la revendication 23, dans lequel l'écharpe est formée d'un matériau perforé.
